# EUROPEAN PATENT SPECIFICATION

(11) **EP 1 348 407 B1**
(45) Date of publication and mention of the grant of the patent: **01.06.2005**
(21) Application number: 03007100.5
(22) Date of filing: 28.03.2003
(51) Int. Cl.: A61F 2/34, A61F 2/30, A61B 17/68

(54) **Acetabular cup for the hip**
Gelenkpfanne für die Hüfte
Coque acétabulaire pour la hanche

(30) Priority: 29.03.2002 IT UD20020073
(43) Date of publication of application: 01.10.2003
(73) Proprietor: LIMA Lto SpA, 33030 Villanova di S. Daniele Del Friuli (UD) (IT)
(72) Inventor: Dalla Pria, Paolo, 33100 Udine (IT)
(74) Representative: Petraz, Gilberto

(56) References cited:
- EP-A- 0 211 169
- EP-A- 0 314 951
- EP-A- 0 368 488
- EP-A- 0 554 210
- EP-A- 0 619 990
- EP-A- 1 062 922
- EP-A- 1 243 234
- WO-A-94/05234
- WO-A-98/04215
- DE-C- 4 133 433
- US-A- 4 840 632
- US-A- 5 360 452
- US-A- 5 593 449

## Description

The present invention concerns an acetabular cup provided with a pin at least partly removable and able to be inserted into a corresponding cavity made in the bone wall of an acetabular seating of a hip, in order to anchor the cup to said bone wall.

The removable pin comprises at least a first coupling portion solid with the acetabular cup and a second portion able to be inserted and clamped inside the acetabular seating and to be associated in removable manner with the first portion, for example by means of conical coupling.

The first coupling portion of the pin has a reduced diameter so that it can be totally inserted inside the axial hole of the second removable portion and completely inscribed therein, thus ensuring optimum conditions of coupling and reciprocal hold.

The acetabular cup according to the invention can be assembled in and released from the acetabular seating simply by coupling or uncoupling the first and second portion of the removable pin.

### BACKGROUND OF THE INVENTION

An acetabular prosthesis is known comprising a semispherical shell, which is attached to the acetabulum of a hip, and provided in a single piece, in correspondence with a central zone of its outer surface, with a fixed conical pin, rather long and hollow inside, which is inserted into a corresponding conical cavity made in the wall of the hip. A screw or other suitable anchoring means is normally inserted and clamped in the cavity of the pin. The pin is used to assemble the acetabular cup with a correct orientation, so that the acetabular cup, both during insertion and also during use, does not rotate or move to an incorrect position. Said pin is particularly useful in cases where there is a lack of bone around the acetabular seating, which makes it difficult and not very reliable to assemble the cup by means of fins, flanges or similar means.

The acetabular cup provided with the pin of a known type has the disadvantage, however, that it is not very practical to assemble in the respective acetabular seating, because it is difficult to insert the pin into the corresponding cavity, due to its length, given the limited space available. Moreover, when the cup has to be removed from the acetabular seating, it is difficult to remove the pin without ruining the bone walls or subjecting them to potentially damaging mechanical stresses.

The Applicant has devised and embodied the present invention to overcome these shortcomings of the state of the art and to obtain other advantages.

US-A-4840632 discloses an acetabular cup according to the preamble of claim 1.

### SUMMARY OF THE INVENTION

The present invention is set forth and characterized essentially in the main claim, while the dependent claims describe other innovative characteristics of the invention.

The purpose of the invention is to achieve an acetabular cup which is easy to attach to, and also to remove from, a corresponding bone seating of a hip.

In accordance with this purpose, an acetabular cup according to the invention comprises at least a shell, able to be inserted and attached in a relative acetabular seating of a hip, and to accommodate a head of a femoral prosthesis.

According to the invention, the acetabular cup comprises pin centering and attachment means able to be inserted and clamped in a cavity made in a bone wall of the acetabular seating. These pin means comprise a first portion, or coupling portion, solid and in a single piece with the outer surface of the shell, and a second portion, or shaft, removable from the first portion, able to be associated with said first portion during the step to assemble the acetabular cup and to be clamped inside said bone cavity.

In a preferential embodiment, the second portion, or shaft, of the pin means is longer than the first coupling portion, and said first portion has a reduced diameter so that it can enter almost completely into the axial hole of the second portion and be totally inscribed inside said hole.

According to a variant, moreover, both portions of the pin means are hollow inside so that they can be passed through by anchoring means to anchor them to the acetabular seating, and are substantially conical in shape, so as to be coupled one to the other by means of conical coupling.

In a preferential embodiment, the second removable portion of the pin means is able to be inserted under pressure into the corresponding bone cavity, and clamped there, by anchoring means, for example a screw or suchlike.

In a variant, said second portion is able to be inserted by screwing into the corresponding bone cavity.

To assemble the acetabular cup, the first portion of the pin means can be selectively constrained to the second, already inserted in the bone cavity, by means of said conical coupling, so that the acetabular cup, solid with said first portion, can be assembled and dis-assembled simply by coupling/uncoupling the two portions of the pin means.

Thanks to the reduced length of the first coupling portion of the pin means, solid with the shell, these operations are extremely easy and quick to perform.

The second portion of the pin means can be kept stably in its seating in the bone cavity also during the possible operations to restore or replace the cup, so as to make the surgeon's task much easier.

According to a variant, on the outside the second removable portion of the pin means comprises fin means, screwing threads or similar means, able to interfere with the wall of the bone cavity to increase the grip and attachment capacity.

### BRIEF DESCRIPTION OF THE DRAWINGS

These and other characteristics of the present invention will become apparent from the following description of a preferential form of embodiment, given as a non-restrictive example with reference to the attached drawings wherein:

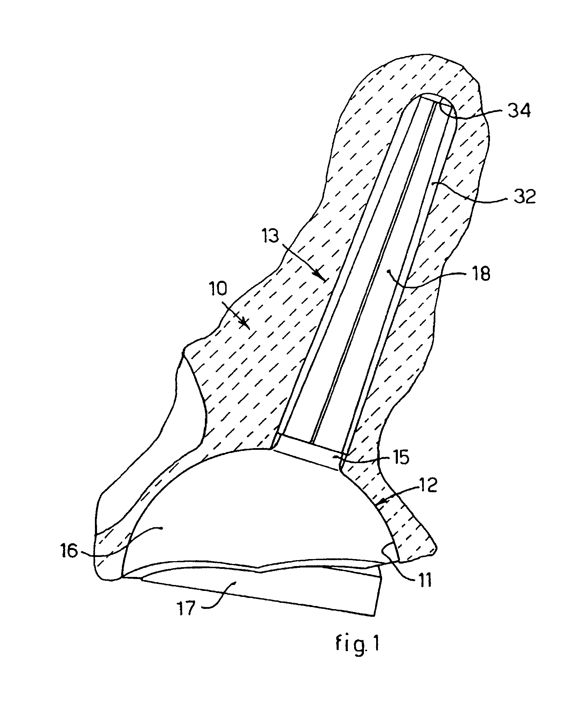

- fig. 1 is a view of an acetabular cup according to the present invention in an assembled condition mounted in a relative acetabular seating;

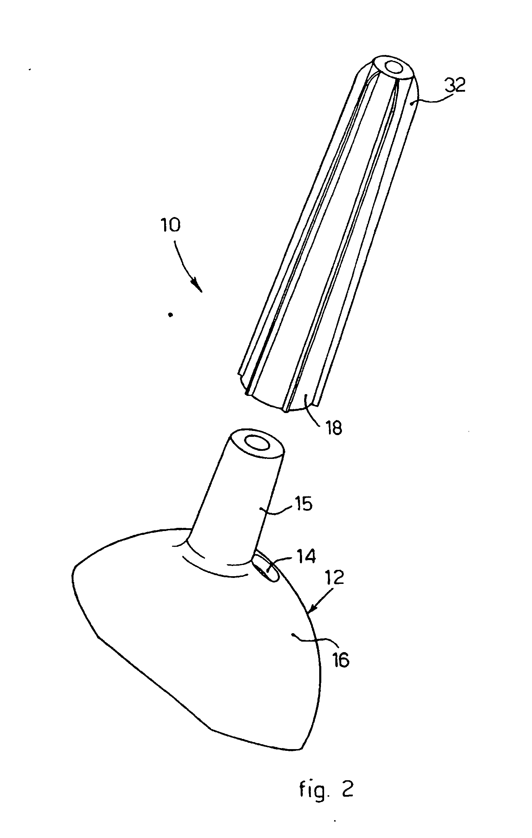

- fig. 2 is an exploded view of the acetabular cup in fig. 1;

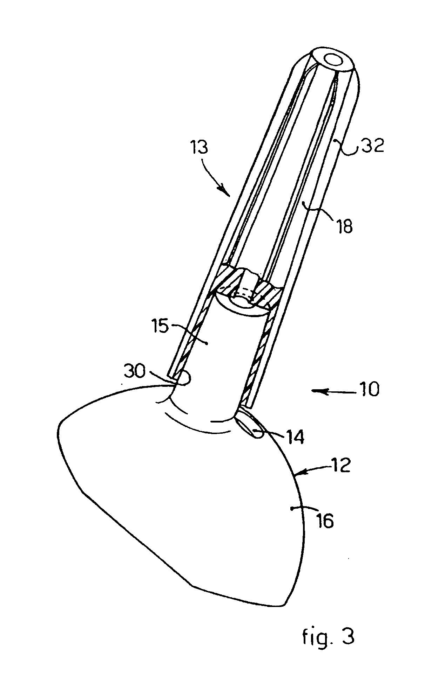

- fig. 3 is a partly sectioned view of the acetabular cup in fig. 1.

### DETAILED DESCRIPTION OF A PREFERENTIAL FORM OF EMBODIMENT OF THE INVENTION

With reference to the attached figures, the acetabular cup 10 according to the present invention comprises pin means 13 associated with the outer surface 16 of a shell 12, able to be assembled in an acetabular seating 11 of a hip. The shell 12 is suitable to accommodate inside itself an insert 17, which functions as a positioning and rotation seating for the head of a femoral prosthesis, not shown here.

The pin 13 comprises a first portion 15, or coupling portion, obtained in a single piece with the outer surface 16 of the shell 12, and a second portion 18, or shaft, removable and able to be coupled with the first portion 15, in this case, by means of a conical coupling.

The second portion 18 is able to be inserted and clamped, through simple pressure, by screwing or by means of auxiliary attachment means, into a corresponding anchoring hole 34 made in the acetabular seating 11.

The elongated shape of the second portion 18 allows it to be manipulated relatively easily, during the pre-assembly step of the cup 10, at the same time ensuring a good anchoring length which improves the stability of the cup 10, when it is assembled.

After the second portion 18 has been mounted in the hole 34, the shell 12 is assembled by coupling the first portion 15 of the pin 13 to the second portion 18.

In this case, the first portion 15 is substantially conical in shape, able to be inserted into the mating conical axial hole 30 of the second portion 18.

The first coupling portion 15 advantageously has a reduced length and a diameter small enough to be completely inserted and inscribed in the conical axial hole 30 of the second portion, or shaft 18. This guarantees a high coupling capacity between the two portions 15 and 18 which improves the conditions of stability, centering and positioning of the cup 10.

The axial hole 30 is through for the whole length of the second portion 18 and has a segment with a reduced diameter with respect to its initial segment where it is able to couple with the first portion 15; according to a variant, the second portion 18 is partly full, at least in its upper part. The first coupling portion 15 is axially holed for the whole of its length, to allow a self-threading attachment screw, not shown in the drawings, to be inserted, which clamps the cup 10 into the corresponding acetabular seating 11.

In this case, the shell 12 has another through hole 14 able to receive a corresponding further attachment screw to attach the cup 10 in the acetabular seating 11.

In a preferential embodiment, the outer surface of the second portion 18 comprises a plurality of fins 32, in this case longitudinal, which increase the interference, and hence the gripping capacity, of the second portion 18 in the anchoring hole 34 made in the bone wall. The fins 32 can be replaced by, or integrated with, screwing threads or similar gripping means.

The acetabular cup 10 according to the present invention can therefore be assembled and dis-assembled simply to/from the acetabular seating 11, without damaging the walls of the bone seating and without requiring complex operations and great ability on the surgeon's part. Moreover, there is the double advantage that a great depth of anchorage is obtained without this depth affecting the simplicity and ease of assembly. In fact, the second portion 18 of the pin 13 is able to be inserted once only, in a definitive and stable manner, by means of simple pressure, into the hole 34. The acetabular cup 10 can then be selectively assembled into, or removed from, the relative acetabular seating 11 by simply coupling or uncoupling the first coupling portion 15 with respect to the second portion 18. The second portion 18, on the contrary, is advantageously kept definitively in the hole 34 of the bone wall, so that the bone wall is never subjected to mechanical stresses if the acetabular cup 10 should be removed.

The reduced diameter of the first coupling portion 15 allows it to be completely inserted inside the conical hole 30 of the second portion 18, to guarantee that the centering and positioning is stable and enduring.

It is clear, however, that modifications and/or additions of parts may be made to the acetabular cup 10 as described heretofore, without departing from the field and scope of the present invention.

## Claims

1. Acetabular cup comprising at least a shell (12), able to be inserted and attached in a relative acetabular seating (11) of a hip, and pin means (13) able to be inserted and clamped in a bone cavity (34) made on a wall of said acetabular seating (11), said pin means (13) comprising a first portion (15), or coupling portion, protruding from an outer surface (16) of said shell (12), and a second portion (18), or shaft, removable with respect to said first portion (15), at least partly holed axially, able to be inserted and clamped inside said bone cavity (34) and to be associated with said first coupling portion (15) in order to assemble said cup in said acetabular seating (11), **characterized in that** the first portion is in a single piece with the outer surface of the shell.

2. Acetabular cup as in claim 1, **characterized in that** said second portion (18) has a greater length than said first coupling portion (15).

3. Acetabular cup as in claim 1 or 2, **characterized in that** said first coupling portion (15) has a sufficiently reduced diameter so that it can enter almost completely into a mating axial hole (30) of said second portion (18) and be completely inscribed in said axial hole (30).

4. Acetabular cup as in any claim hereinbefore, **characterized in that** said first coupling portion (15) is at least partly hollow inside so as to be passed through by anchoring means to anchor said first coupling portion (15) to said acetabular seating (11).

5. Acetabular cup as in claim 4, **characterized in that** said first coupling portion (15) and said second portion (18) are at least partly substantially conical in shape so as to be coupled with each other by means of conical coupling.

6. Acetabular cup as in any claim hereinbefore, **characterized in that** said second portion (18) is able to be inserted and clamped under pressure in said bone cavity (34).

7. Acetabular cup as in any claim from 1 to 5 inclusive, **characterized in that** said second portion (18) is able to be clamped through screwing into said bone cavity (34).

8. Acetabular cup as in any claim hereinbefore, **characterized in that**, on the outside, said second portion (18) has fin means (32), screwing threads or other means able to increase interference and the attachment capacity of said pin means (13) in said bone cavity (34).

## Patentansprüche

1. Hüftgelenkpfanne umfassend: mindestens eine Schale (12), die in einem entsprechenden Hüftgelenkssitz (11) einer Hüfte eingesetzt und angebracht werden kann, und eine Stifteinrichtung (13), die in einem Knochenhohlraum (34) eingesetzt und festgeklemmt werden kann, der in einer Wand des Hüftgelenksitzes (11) hergestellt ist, wobei die Stifteinrichtung (13) umfasst: einen ersten Teil (15) oder Kopplungsteil, der von einer äußeren Oberfläche (16) der Schale (12) vorsteht, und einen zweiten Teil (18) oder Schaft, der in Bezug zu dem ersten Teil (15) entfernbar ist, mindestens teilweise axial ausgehöhlt ist, im Innern des Knochenhohlraums (34) eingesetzt und festgeklemmt werden kann und mit dem ersten Kopplungsteil (15) verbindbar ist, um die Pfanne in dem Hüftgelenksitz (11) zu montieren, **dadurch gekennzeichnet, dass** der erste Teil einteilig mit der äußeren Oberfläche der Schale ist.

2. Hüftgelenkpfanne nach Anspruch 1, **dadurch gekennzeichnet, dass** der zweite Teil (18) eine größere Länge aufweist als der erste Kopplungsteil (15).

3. Hüftgelenkpfanne nach Anspruch 1 oder 2, **dadurch gekennzeichnet, dass** der erste Kopplungsteil (15) einen ausreichend verringerten Durchmesser aufweist, so dass er nahezu vollständig in ein passendes axiales Loch (30) des zweiten Teils (18) eintreten kann und vollständig in dem axialen Loch (30) einbeschrieben wird.

4. Hüftgelenkpfanne nach einem vorangehenden Anspruch, **dadurch gekennzeichnet, dass** der erste Kopplungsteil (15) mindestens teilweise im Innern hohl ist, so dass er von Verankerungseinrichtungen durchquert werden kann, um den ersten Kopplungsteil (15) mit dem Hüftgelenksitz (11) zu verankern.

5. Hüftgelenkpfanne nach Anspruch 4, **dadurch gekennzeichnet, dass** der erste Kopplungsteil (15) und der zweite Teil (18) mindestens teilweise im Wesentlichen konusförmig sind, so dass sie mittels einer Konuskopplung miteinander gekoppelt werden.

6. Hüftgelenkpfanne nach einem vorangehenden Anspruch, **dadurch gekennzeichnet, dass** der zweite Teil (18) unter Druck in den Knochenhohlraum (34) eingesetzt und festgeklemmt werden kann.

7. Hüftgelenkpfanne nach einem Anspruch von 1 bis 5 einschließlich, **dadurch gekennzeichnet, dass** der zweite Teil (18) durch Einschrauben in den Knochenhohlraum (34) festgeklemmt werden kann.

8. Hüftgelenkpfanne nach einem vorangehenden Anspruch, **dadurch gekennzeichnet, dass** auf der Außenseite der zweite Teil (18) Rippeneinrichtungen (32), Schraubgewinde oder andere Einrichtungen aufweist, die einen Eingriff und das Anbringvermögen der Stifteinrichtung (13) in dem Knochenhohlraum (34) verstärken können.

## Revendications

1. Coque acétabulaire comprenant au moins une coquille (12) en mesure d'être insérée et attachée dans une assise acétabulaire relative (11) d'une hanche et d'un moyen formant broche (13) en mesure d'être insérée et clampée dans une cavité osseuse (34), constituée d'une paroi de ladite assise acétabulaire (11), ledit moyen formant broche (13) comprenant une première partie (15) ou une partie de couplage formant une protubérance depuis une surface externe (16) de ladite coquille (12) et une seconde partie (18), ou tige, amovible concernant ladite première partie (15), au moins partiellement trouée de façon axiale en mesure d'être insérée et clampée à l'intérieur de ladite cavité osseuse (34) et d'être associée avec ladite première partie de couplage (15) afin d'assembler ladite coquille dans ladite assise acétabulaire (11), **caractérisée en ce que** la première position se trouve dans une pièce unique avec la surface externe sur la coquille.

2. Coque acétabulaire selon la revendication 1,
**caractérisée en ce que** ladite seconde partie (18) présente une longueur supérieure à ladite première partie de couplage (15).

3. Coque acétabulaire selon l'une quelconque des revendications 1 ou 2, **caractérisée en ce que** ladite première partie de couplage (15) présente un diamètre suffisamment réduit de sorte à pouvoir entrer quasiment complètement dans le trou axial d'intégration (30) de ladite seconde partie (18) et être inscrite complètement dans ledit trou axial (30).

4. Coque acétabulaire selon l'une quelconque des revendications précédentes, **caractérisée en ce que** ladite première partie de couplage (15) est au moins partiellement creuse à l'intérieur de sorte à être traversée par un moyen formant ancrage pour ancrer ladite première partie de couplage (15) à ladite assise acétabulaire.(11).

5. Coque acétabulaire selon la revendication 4, **caractérisée en ce que** ladite première partie de couplage (15) et ladite seconde partie (18) sont au moins substantiellement coniques en partie en termes de forme de sorte à être couplées l'une avec l'autre par le biais d'un moyen formant couplage conique.

6. Coque acétabulaire selon l'une quelconque des revendications précédentes, **caractérisée en ce que** ladite seconde partie (18) est en mesure d'être insérée et clampée sous pression dans ladite cavité osseuse (34).

7. Coque acétabulaire selon l'une quelconque des revendications 1 à 5 incluse, **caractérisée en ce que** ladite seconde partie (18) est en mesure d'être clampée par vissage dans ladite cavité osseuse (34).

8. Coque acétabulaire selon l'une quelconque des revendications précédentes, **caractérisée en ce que** sur la partie externe, ladite seconde partie (18) présente un moyen formant ailette (32), des filetages de vissage ou d'autres moyens en mesure d'augmenter l'interférence et la capacité de fixation dudit moyen formant broche (13) dans ladite cavité osseuse (34).
